# EUROPÄISCHE PATENTSCHRIFT

(11) **EP 2 641 554 B1**
(45) Veröffentlichungstag und Bekanntmachung des Hinweises auf die Patenterteilung: **24.08.2016**
(21) Anmeldenummer: 13159778.3
(22) Anmeldetag: 18.03.2013
(51) Int. Cl.: A61B 18/14, A61M 25/00

(54) **Endoskopisches Chirurgieinstrument**
Endoscopic surgical instrument
Instrument chirurgical endoscopique

(30) Priorität: 19.03.2012 DE 102012102271
(43) Veröffentlichungstag der Anmeldung: 25.09.2013
(73) Patentinhaber: Ovesco Endoscopy AG, 72074 Tübingen (DE)
(72) Erfinder: Anhöck, Gunnar, 72764 Reutlingen (DE); Menge, Sebastian, 72074 Tübingen (DE); Ho, Chi-Nghia, 70191 Stuttgart (DE); Schurr, Marc O., Prof. Dr., 72072 Tübingen (DE)
(74) Vertreter: Winter, Brandl, Fürniss, Hübner, Röss, Kaiser, Polte - Partnerschaft mbB

(56) Entgegenhaltungen:
- EP-A1- 2 156 801
- US-A1- 2009 254 083
- US-A1- 2012 035 605
- US-B1- 6 295 990

## Beschreibung

Die vorliegende Erfindung betrifft ein endoskopisches Chirurgieinstrument der TFT-Bauart und insbesondere ein endoskopisches Chirurgieinstrument mit einer Fluid umspülten HF-Elektrode.

### Hintergrund der Erfindung

In der modernen minimalinvasiven Chirurgie werden zunehmend chirurgische Instrumente mit TFT-Bauweise eingesetzt, bei denen HF-Elektroden in mono- oder bipolarer Anordnung mit einem HF-Strom beaufschlagt werden, um ein Organgewebe zu zerschneiden und/oder zu verschweißen/versiegeln. Um den Zugangskanal für das chirugische Instrument möglichst klein zu halten und dennoch eine ausreichend gute Sicht zur Operationsstelle zu gestatten, ist es wichtig, die Operationsstelle möglichst sauber und frei von Gewebeteilchen und/oder Blut zu halten. In sofern kommt dem Spülen der Operrationsstelle insbesondere bei der minimalinvasiven Chirurgietechnik immer größere Bedeutung zu.

### Stand der Technik

Aus der EP 1 834 598 A1 und EP 2 156 801 ist ein endoskopisches Chirurgieinstrument der vorliegenden Gattung bekannt. Dieses hat einen Zuführschaft, an dessen proximalem (körperabgewandtem) Ende eine Handhabe/Instrumentengriff angeordnet ist und an dessen distalem (körperzugewandtem) Ende ein stopfenförmiges Einsatzteil vorgesehen ist, der in den Zuführschaft eingesetzt ist. Das Einsatzteil hat ein zentrales Durchgangsloch sowie zumindest einen dezentralen Spül-Durchgangskanal.

Im Zuführschaft ist eine nadel- oder stiftförmige HF-Elektrode axial gleitgeführt, die über eine Stromleitung innerhalb des Zuführschafts mit dem Instrumentengriff verbunden ist. Die HF-Elektrode steckt hierbei in einer Führungshülse, welche am Zuführschaft gleitend bewegbar ist und in welcher eine Anzahl von dezentralen Axial-Durchgangsbohrungen ausgeformt sind. Diese Führungshülse dient jedoch nicht nur als distales Gleitlager für die HF-Elektrode, sondern auch als ein Axialanschlag zu Begrenzung der axialen Vorschubbewegung der HF-Elektrode. In anderen Worten ausgedrückt kommt die Führungshülse beim Vorschieben der HF-Elektrode durch die zentrale Durchgangsbohrung des Einsatzteils hindurch axial mit diesem in Anlage und stoppt dabei diese Vorschubbewegung.

Obgleich durch diese Konstruktion eine Zufuhr an Spülmedium zur Operationsstelle möglich ist, so muss das Spülmedium sowohl die Durchgangskanäle in der Führungshülse wie auch im Einsatzteil passieren, welche ein großes Strömungshindernis darstellen. Der Fluiddruck ist daher an der Operationsstelle nicht sehr hoch.

### Kurzfassung der Erfindung

Angesichts dieser Problematik ist es die Aufgabe der vorliegenden Erfindung, ein endoskopisches Chirurgieinstrument bereit zu stellen, das eine gesteigerte Funktionalität aufweist. Ein besonderes Ziel ist es, die Spüleigenschaft des endoskopischen Chirurgieinstruments insbesondere im Fall einer TFT-Bauweise zu verbessern.

Diese Aufgabe wird durch ein endoskopisches Chirurgieinstrument mit den Merkmalen des Patentanspruchs 1 gelöst. Vorteilhafte Ausgestaltungen der Erfindung sind Gegenstand der Unteransprüche.

Der Grundgedanke der vorliegenden Erfindung besteht demzufolge gemäß einem Aspekt in der Bereitstellung eines endoskopischen Chirurgieinstruments mit einer vorzugsweise schlauchförmigen Außenhülle, in der eine vorzugsweise ebenfalls schlauchförmige Innenhülle relativ verschiebbar gelagert ist, an deren distalem Endabschnitt ein hülsen- oder schaftförmiger Instrumentenkopf ausgebildet oder montiert ist, der in einer maximal vorgeschobenen Position distal aus der Außenhülle axial herausragt. In dem Instrumentenkopf ist eine HF-Elektrode in elektrisch isolierter Weise fixiert, deren elektrische Zuleitung durch die Innenhülle geführt ist und die in Axialrichtung über das distale Ende des Instrumentenkopfs frei vorragt. Dabei ist der Instrumentenkopf soweit in die Außenhülle axial zurückziehbar, dass die HF-Elektrode vollständig axial hinter (in proximaler Richtung) dem distalen Ende der Außenhülle zu liegen kommt, also vollständig von der Außenhülle umgeben wird. Des weiteren ist in dem Instrumentenkopf zumindest ein einzelner (oder mehrere) axial sich erstreckender sowie an beiden axialen Instrumentenkopfenden sich öffnender Spülkanal ausgeformt, der gleich zeitig auch die Austrittsöffnung für das Spülmedium aus dem Chirurgieinstrument darstellt, welche somit zusammen mit der HF-Elektrode axial mitbewegt wird und daher die Relativposition zur HF-Elektrode beibehält.

Auf diese Weise kann der Spülstrahl immer optimal zur HF-Elektrode geführt werden, wodurch sich das Spülergebnis insgesamt verbessert. Das des Weiteren nur ein Spülkanal in Axialrichtung gesehen passiert werden muss, reduziert sich der Strömungswiderstand, sodass der Spüldruck ansteigt.

Grundsätzlich ist es bei dem vorliegenden Instrument so, dass die HF-Elektrode im Instrumentenkopf fixiert ist - also nicht axial relativbewegt werden kann. Satt dessen wir der Instrumentenkopf innerhalb der äußeren Hülle axial gleitgeführt. Der grundlegende technische Hintergrund zu dieser Konstruktion basiert auf der folgenden Überlegung:

Die HF-Elektrode muss in vorteilhafter Weise im Wesentlichen exakt positionierbar sein, wofür sie in einer Lagerung geführt sein sollte: Wenn daher beispielsweise ein Endoskopschaft als Bestandteil eines darin eingeführten Chirurgieinstruments betrachtet wird und der Instrumentenschaft mit der erfindungsgemäßen Innenhülle gleichgesetzt wird, dann müsste der Endoskopschaft (bzw. dessen distaler Endabschnitt) eine solche Führungsfunktion übernehmen. Es ist aber bei Endoskopen und darin eingeführten Chirurgieinstrumenten so, dass die Innenhülle (das Chirurgieinstrument) aus dem Arbeitskanal des Endoskops in Richtung distal ungeführt vorgeschoben wird. Ein solches "Herausschlabbern" des Instruments aus dem Endoskop hat sicherlich eine Führungsqualität, die es nicht erlaubt, die HF-Elektrode mittels des Endoskopschafts bzw. der Einführhilfe selbst exakt zu positionieren/führen. In anderen Worten ausgedrückt dient ein Endoskopschaft ausschließlich dazu, das Chirurgieinstrument in ein Holorgan einzuführen, dann wird das Chirurgieinstrument aus dem Endoskopschaft axial herausgeschoben und schließlich wird die HF-Nadel/Elektrode frei vorgeschoben, wobei dieses Verschieben innerhalb des Chirurgieinstruments in der Regel durch einen Einsatz geführt ist. Das bedeutet, dass die HF-Nadel/Elektrode zwangsläufig radial gelagert/abgestützt sein muss.

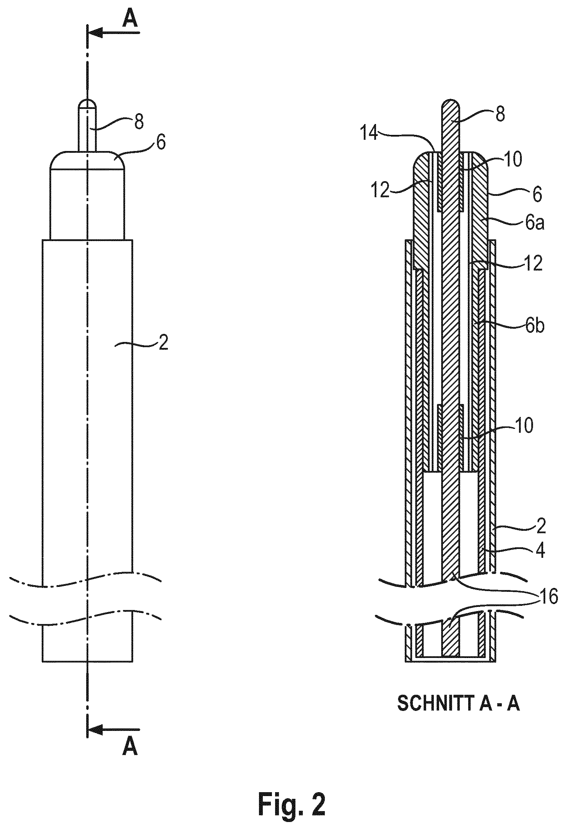

Es ist bekannt, dass diese Abstützung mittels des in der Instrumentenhülle (Innenhülle) fixierten Einsatzes erfolgt, der ein zentrales Durchgangsloch hat, in das die HF-Nadel/Elektrode gleitend eingeführt ist. Bei der vorliegenden Erfindung aber ist es genau umgekehrt. D. h., erfindungsgemäß sitzt die HF-Nadel/Elektrode fest in einem zentralen Durchgangsloch eines Gleitschuhs, nämlich des Endoskopkopfs, der an seinem Außenradius an einer Außenhülle des Chirurgieinstruments gleitend geführt ist und diesen Gleitführungskontakt in allen Betriebspositionen auch beibehält, wie dies nachfolgend anhand Fig. 2 beschrieben wird. Darüber hinaus ist es bevorzugt vorgesehen, dass der Endoskopkopf in aktiver Position des Chirurgieinstruments (nur) teilweise aus der schlauchförmigen Außenhülle geschoben ist und im übrigen auch noch Gleitkontakt hat, und damit geführt bleibt.

Schließlich ist es bevorzugt vorgesehen, dass zur Vermeidung von Verkanten des Instrumentenkopfs dieser größer sein soll als der Durchmesser der Außenhülle, in welcher er gleitgeführt ist.

Dabei kann die Gleitführung erfindungsgemäß fluiddicht ausgeführt sein.

Vorteilhaft ist es, wenn die proximale Spülkanalöffnung unmittelbar in den Innenraum der Innenhülle mündet, um mit einem durch die Innenhülle geförderten Spülmedium beaufschlagbar zu sein. Dadurch kann der Strömungsquerschnitt innerhalb des Instrumentenschafts (Außen- und Innenhülle) vergrößert werden.

Eine weitere vorteilhafte Ausbildung der Erfindung sieht vor, dass der Instrumentenkopf vorzugsweise fluiddicht an der Außenhülle gleitgeführt ist. Dadurch können auf zusätzliche Lagermaßnahmen verzichtet werden, sodass sich die Gesamtkonstruktion des Chirurgieinstruments vereinfacht.

Ein zusätzlicher oder alternativer Aspekt der vorliegenden Erfindung sieht vor, dass der hülsenförmige Instrumentenkopf einen distalen Axialabschnitt mit großem Außendurchmesser als Gleitführung an der Außenhülle und einen proximalen Axialabschnitt mit kleinem Außendurchmesser als fluiddichtenden Aufsteck- oder Aufschraubsockel für die Innenhülle hat. Dadurch erhält der Instrumentenkopf eine bestimmte axiale Länge, die eine verkantenfreie Führung innerhalb der Außenhülle ermöglicht. Diese Konstruktion bildet somit die Voraussetzung dafür, dass kein weiteres Axial-Gleitlager vorgesehen werden muss, durch welches das Spülmedium hindurchgeführt werden muss.

Vorteilhaft ist es, wenn die vorzugsweise fluiddichte Fixierung der HF-Elektrode im Instrumentenkopf mittels zumindest einer Klemmhülse, vorzugsweise zweier axial beabstandeter Klemmhülsen erfolgt, die um die HF-Elektrode geführt und stopfenartig in den hülsenförmigen Instrumentenkopf eingesetzt ist/sind. Dadurch wird zum Einen eine ausreichende Fixierung der HF-Elektrode mit dem Instrumentenkopf erreicht und zum Anderen die Innenhülle fluiddicht verschlossen, sodass diese Als Leitung für das Spülmedium nutzbar ist.

Wie vorstehend bereits angedeutet wurde ist es weiter vorteilhaft, wenn der hülsenförmige Instrumentenkopf mit einer Vorschubkraft als Schubkraft über die Innenhülle und/oder die elektrische Zuleitung beaufschlagbar ist, um die HF-Elektrode und zumindest teilweise auch den hülsenförmigen Instrumentenkopf aus dem distalen Ende der Außenhülle vorzuschieben und/oder in die Außenhülle zurückzuziehen, soweit, dass die HF-Elektrode in zurückgezogener Position proximal vollständig (einschließlich der freien Elektrodenspitze) hinter dem distalen Ende der Außenhülle zu liegen kommt. In dieser zurückgezogenen Position wird somit die HF-Elektrode vollständig von der Außenhülle (aus elektrisch isolierendem Material) umschlossen und kann so kein Organgewebe beschädigen.

### Figurenbeschreibung

Die Erfindung wird nachstehend anhand eines bevorzugten Ausführungsbeispiels unter Bezugnahme auf die begleitenden Zeichnungen näher erläutert.
Fig. 1 zeigt die Perspektivenansicht eines distalen Endabschnitts eines endoskopischen Chirurgieinstruments der TFT-Bauweise gemäß einem bevorzugten Ausfürhungsbeispiel der Erfindung,
Fig. 2 zeigt die Seiten- sowie zugehörige Längsschnittansicht des distalen Endabschnitts gemäß der Fig. 1 in einer aktivierten (vorgeschobenen) HF-Elektrodenposition und
Fig. 3 zeigt die Seiten- sowie zugehörige Längsschnittansicht des distalen Endabschnitts gemäß der Fig. 1 in einer de-aktivierten (vorgeschobenen) HF-Elektrodenposition.

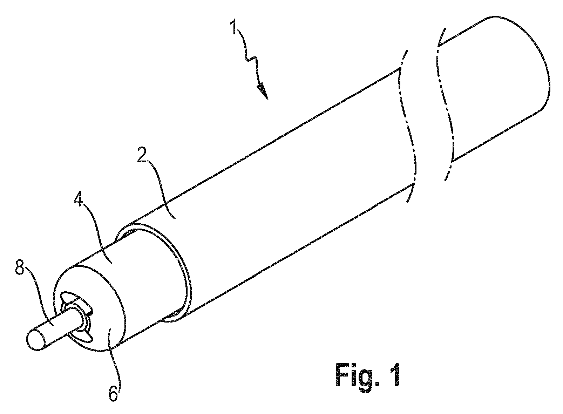

Gemäß der Fig. 1 hat das endoskopische Chirurgieinstrument der TFT-Bauweise eines bevorzugten Ausführungsbeispiels der Erfindung einen vorzugsweise biegeflexiblen Schaft 1, an dessen proximalem Ende ein Instrumentengriff oder eine Betätigungseinrichtung (nicht weiter dargestellt) angeordnet ist. Als "biegeflexibel" ist eine Funktionseigenschaft des Instrumentenschafts zu verstehen, die es dem Instrument erlaubt/ermöglicht, den Windungen eines Hohlorgans vorzugsweise eines menschlichen Körpers, wie beispielsweise der Darm, die Speiseröhre, ein Blutgefäß, etc. zu folgen, ohne das Hohlorgan übergebührlich zu deformieren. D.h. die Steifigkeit des Instrumentenschafts zumindest in Biegerichtung sollte kleiner sein, als das zu behandelnde Organgewebe, sodass bei der Penetrationsbewegung nicht das Hohlorgan sondern im Wesentlichen der Instrumentenschaft deformiert/gebogen wird.

Der Instrumentenschaft 1 besteht vorliegend aus einer schlauchförmigen (ersten) Außenhülle 2 (beispielsweise aus einem Silikon- oder PVC-Material oder einem anderen biokompatiblen flexiblen Material), in der eine ebenfalls schlauchförmige (zweite, separate) Innenhülle 4 axial-gleitgeführt ist, welche aus einem gleichen bzw. ähnlichen Material gefertigt sein kann. Die Außen- und/oder Innenhülle 2, 4 kann eine Einlage beispielsweise aus einem Gewebe, einer Spirale oder dergleichen Aussteifung aufweisen, um die Steifigkeit in Axialrichtung bei hoher Biegeflexibilität zu erhöhen und/oder die Aufweitbarkeit im Fall eines Innendruckanstiegs zu verringern.

Am in der Fig. 1 gezeigten distalen Endabschnitt des Chirurgieinstruments hat die Innenhülle 4 einen Instrumentenkopf 6, der den Innenraum der Innenhülle 4 mit Ausnahme zumindest eines Spülkanals (wird nachfolgend noch näher beschrieben) axial verschließt und der eine HF-Elektrode 8 (fest) vorzugsweise zentral trägt, die axial aus dem Instrumentenkopf 6 herausragt, um eine HF-Elektrodenschneide auszuformen.

In der Fig. 2 ist das erfindungsgemäße Chirurgieinstrument im Seiten- und Längsschnitt dargestellt. Demzufolge besteht der Instrumentenkopf 8 aus einem hülsenförmigen Bolzen oder Schaftstück vorzugsweise aus einem keramischen Material mit zwei axial beabstandeten (angrenzenden) Funktionsabschnitten 6a, 6b unterschiedlicher Außendurchmesser. Ein distaler Funktionsabschnitt 6a mit großem Außendurchmesser ist dafür vorgesehen und dazu angepasst, an der Innenseite der schlauchförmigen Außenhülle 2 zu gleiten. Demzufolge ist der Außendurchmesser des distalen Funktionsabschnitts 6a des Instrumentenkopfs 6 unter Ausbildung eines kleinen Spiels (Spalts) nur geringfügig kleiner als der Innendurchmesser der Außenhülle 2, sodass im günstigsten Fall sogar eine Dichtwirkung zwischen dem Instrumentenkopf 6 und der Außenhülle 2 erreicht wird. Ein proximaler Funktionsabschnitt 6b mit (gegenüber dem distalen Funktionsabschnitt 6a) kleinem Außendurchmesser ist dafür vorgesehen und dazu angepasst, distal sowie axial in die schlauchförmige Innenhülle 4 fluiddicht eingesetzt (eingepresst, geklebt, geschraubt, etc.) zu werden. Die Gesamtlänge des Instrumentenkopfs (hülsenförmigen Bolzens) 6 ist vorzugsweise so gewählt, dass dieser bei einer Axialverschiebung längs der Außenhülle 2 in dieser nicht oder nur schwer verkanten kann. D.h. die Gesamtlänge des Instrumentenkopfs (hülsenförmigen Bolzens) 6 ist (wesentlich) größer als der Durchmesser der Außenhülle 2.

Der hülsenförmige Bolzen 6 hat eine durchgehende, vorzugsweise zentrale Axialbohrung, in die die eine HF-Elektrode 8 eingeführt ist, derart, dass diese um eine vorbestimmte Länge über die distale Stirnseite des Bolzens 6 vorragt und dabei die HF-Elektrodenschneide ausbildet. Im Konkreten wird die HF-Elektrode 8 durch einen Draht oder Stift aus elektrisch leitendem Material gebildet, der in der Axialbohrung des Bolzens 6 vorzugsweise an beiden axialen Bolzenenden mittels den Draht 8 umgebender Klemmhülsen 10 fixiert ist, die hierfür in die Axialbohrung eingepresst sind. Der Bolzen 6 ist ferner an seinem proximalen Funktionabschnitt 6b in die schlauchförmige Innenhülle 4 axial sowie distal dichtend eingesetzt, wie dies vorstehend bereits angedeutet wurde, wobei die Innenhülle 4 soweit über den proximalen Funktionsabschnitt 6b in Richtung distal gezogen ist, bis diese am distalen Funktionsabschnitt 6a (mit größerem Außendurchmesser) anschlägt.

Der hülsenförmige Bolzen 6 hat des Weiteren zumindest einen, vorzugsweise eine Anzahl gleichmäßig winkelbeabstandeter axialer Durchgangskanäle (Bohrungen) 12, welche die zentrale HF-Elektrode 8 auf einer Kreisbahn umgebend angeordnet sind und Spülkanäle ausbilden. Diese Spülkanäle 12 öffnen sich an der proximalen Stirnseite des Bolzens 6 in den Innenraum der schlauchförmigen Innenhülle 4 und bilden an der proximalen Stirnseite des Bolzens 6 Spülmedium - Auslassöffnungen (-düsen) 14 des Chirurgieinstruments. D.h. diese Spülmedium -Auslassöffnungen 14 sind die distal vordersten (letzten) Instrumenten-Spülöffnungen, von wo aus ein Spülmedium (Spülflüssigkeit) direkt auf eine Operationsstelle/HF-Elektrode gesprüht wird. Dieses Spülmedium wird hierfür durch die schlauchförmige Innenhülle 4 mit vorbestimmtem Druck über die gesamte Instrumentenschaftlänge gepresst.

Wie aus der Fig. 2 auch zu entnehmen ist, verläuft innerhalb der schlauchförmigen Innenhülle 4 auch eine elektrische Zuleitung 16 für die wahlweise Beaufschlagung der HF-Elektrode 8 mit einem elektrischen Strom. Die schlauchförmige Innenhülle 4 und/oder die Zuleitung 16 dienen dabei zusätzlich zur Übertragung einer Zug-/Druckkraft auf den Instrumentenkopf, letztlich umfassend den hülsenförmigen Bolzen 6 und die darin fixierte HF-Elektrode 8, um den Instrumentenkopf, d.h. den Bolzen 6 und die HF-Elektrode 8 längs der schlauchförmigen Außenhülle 2 zu bewegen.

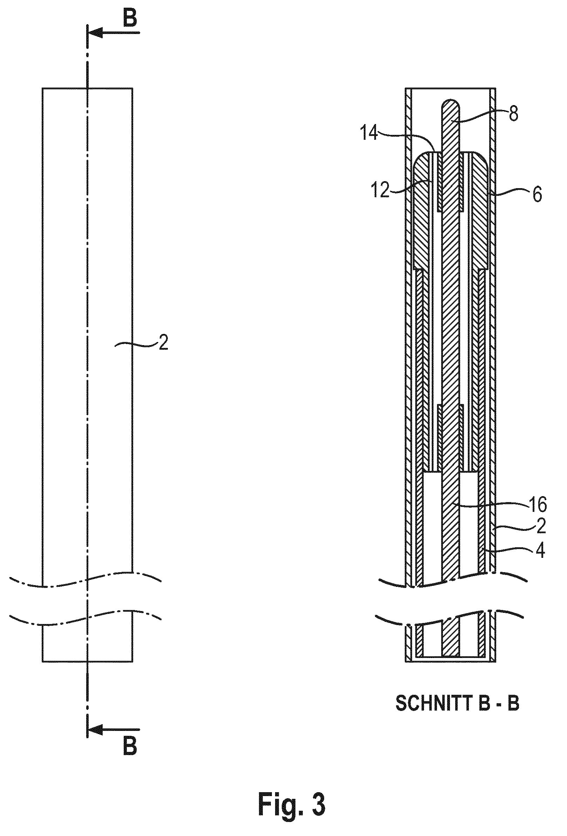

Die Funktionsweise des erfindungsgemäßen Chirurgieinstruments wird nachfolgend anhand der Fig. 2 und 3 beschrieben.

In der Fig. 2 ist das erfindungsgemäße Chirurgieinstrument in aktivierter Position gezeigt. Demzufolge ist der Instrumentenkopf 6 insbesondere der frei vorragende Teil der HF-Elektrode 8 (Schneide-Abschnitt) zur Gänze aber auch der hülsenförmige Bolzen 6 zumindest teilweise axial aus der schlauchförmigen Außenhülle 2 geschoben. Zur Vermeidung von Gewebeverletzungen ist der Bolzen 6 zumindest in dem nach Außen exponierten Bereich abgerundet. Die Auslassöffnungen oder Sprühöffnungen 14 der Spülkanäle 12 sind zusammen mit der im Instrumentenkopf 6 fest fixierten HF-Elektrode 8 nach distal mitbewegt worden und behalten somit ihre Relativlage zur HF-Elektrode 8, insbesondere deren freie Elektrodenspitze bei. Damit ist in jeder Axialposition der HF-Elektrode 8 relativ zur Außenhülle 2 immer die optimale Spülwirkung erzielbar.

In der Fig. 3 ist das erfindungsgemäße Chirurgieinstrument in de-aktivierter Position dargestellt. In diesem Fall ist der Instrumentenkopf 6 vollständig in die schlauchförmige Außenhülle 2 in Richtung proximal zurückgezogen, soweit, dass auch die Elektrodenspitze (proximal) hinter der distalen Stirnkante der Außenhülle 2 liegt und somit vollständig von dieser umschlossen wird.

In dieser Stellung kann die HF-Elektrode auch bei unbeabsichtigter Bestromung kein Organgewebe mehr beschädigen. In dieser Position sind auch die Spülaustrittsöffnungen 14 des Chirurgieinstruments zusammen mit dem Instrumentenkopf 6 in die Außenhülle 2 nach proximal zurück gezogen und können so nicht mehr von Körperflüssigkeit oder Gewebe verstopft oder kontaminiert werden.

Offenbart wird zusammenfassend ein endoskopisches Chirurgieinstrument mit einer einen Instrumentenschaft ausbildenden schlauchförmigen Außenhülle 2, in der eine ebenfalls schlauchförmige Innenhülle 4 relativ verschiebbar gelagert ist, an deren distalem Endabschnitt ein hülsen- oder schaftförmiger Instrumentenkopf 6 ausgebildet oder montiert ist. In dem Instrumentenkopf 6 ist eine vorzugsweise stift- oder nadelförmige HF-Elektrode 8 in elektrisch isolierter Weise fixiert, deren elektrische Zuleitung 16 durch die Innenhülle 4 längs geführt ist und die in Axialrichtung über das distale Ende des Instrumentenkopfs 6 frei vorragt. Im Instrumentenkopf 6 ist ferner zumindest ein (vorzugsweise mehrere gleichmäßig Winkel beabstandete) axial sich erstreckender, sowie an beiden Instrumentenkopfenden sich öffnender Spülkanal 14 ausgeformt, der am distalen Ende des Instrumentenkopfs 6 die eigentliche (distal letzte) Spülmittel -Austrittöffnung des Chirurgieinstruments für ein Spülmedium ausbildet. Wird folglich die HF-Elektrode 8 (zwangsläufig) teilweise zusammen mit dem Instrumentenkopf 6 axial aus der Außenhülle 2 nach Vorn in Richtung distal herausgeschoben, wandert die Spülmediums-Austrittsöffnung 14 mit der HF-Elektrode 8 mit, sodass der Abstand und die Ausrichtung zwischen der zumindest einen Austrittsöffnung 14 und HF-Elektrode 8 unverändert bleibt. Somit kann in jeder Vorschubposition der HF-Elektrode 8 bezüglich der Außenhülle 2 ein maximales Spülergebnis erreicht werden.

## Patentansprüche

1. Endoskopisches Chirurgieinstrument, welches dazu angepasst ist, in einen Arbeitskanal eines Endoskops eingeführt zu werden, mit:
einer Außenhülle (2), in der eine Innenhülle (4) relativ verschiebbar gelagert ist,
einem hülsenförmigen Instrumentenkopf (6), der am distalen Endabschnitt der Innenhülle (4) ausgebildet oder fest montiert ist, wobei
der Instrumentenkopf (6) einen Spülkanal (12) aufweist, der sich an beiden Axialenden des Instrumentenkopfes (6) öffnet,
einer HF-Elektrode (8), die in dem Instrumentenkopf (6) in elektrisch isolierter Weise aufgenommen ist, wobei
die Elektrode (8) eine elektrische Zuleitung (16) aufweist, die innerhalb der Innenhülle (4) längs verlegt ist und wobei
die HF-Elektrode (8) zusammen mit dem Instrumentenkopf (6) und der elektrischen Zuleitung (16) in Axialrichtung über das distale Ende der Außenhülle (2) hinaus verschiebbar ist, wobei
die Relativstellung zwischen der HF-Elektrode (8) und einer von dem zumindest einen Spülkanal (12) unmittelbar gebildeten, distal letzten Instrumenten-Spülmediumsaustrittsöffnung (14) konstant bleibt,
**dadurch gekennzeichnet, dass**
die HF-Elektrode (8) unbeweglich in dem Instrumentenkopf (6) derart fixiert ist, dass sie in jeder Relativ-Betriebsposition zur Außenhülle (2) in Axialrichtung über das distale Ende des Instrumentenkopfs (6) grundsätzlich frei vorragt und dass der Instrumentenkopf (6) zusammen mit der Innenhülle (4) in jeder Relativ-Betriebsposition zur Außenhülle (2) zumindest teilweise in dieser, fluiddicht, gleitgeführt verbleibt, derart, dass der Instrumentenkopf (6) einschliesslich der HF-Elektrode (8) und der elektrischen Zuleitung (16) in Axialrichtung aus einer diese von der Außenhülle (2) vollständig umgebenden Position über das distale Ende der Außenhülle (2) hinaus verschiebbar ist und dass sich der Spülkanal (12) an der proximalen Stirnseite des Instrumentenkopfs (6) in den Innenraum der schlauchförmigen Innenhülle (4) öffnet und an der distalen Stirnseite des Instrumentenkopfs (6) die Instrumenten-Spülmediumsaustrittsöffnung (14) bildet.

2. Endoskopisches Chirurgieinstrument nach Anspruch 1, **dadurch gekennzeichnet, dass**
der Spülkanal (12) mit einem durch die Innenhülle (4) geförderten Spülmedium beaufschlagbar ist.

3. Endoskopisches Chirurgieinstrument nach Anspruch 1 oder 2, **dadurch gekennzeichnet, dass** der hülsenförmige Instrumentenkopf (6) einen distalen Axialabschnitt (6a) mit großem Außendurchmesser hat, der unmittelbar an der Außenhülle (2) in Gleitanlage ist und einen proximalen Axialabschnitt (6b) mit kleinem Außendurchmesser als fluiddichtenden Aufsteck- oder Aufschraubsockel für die Innenhülle (4) hat.

4. Endoskopisches Chirurgieinstrument nach einem der vorstehenden Ansprüche, **dadurch gekennzeichnet, dass** die vorzugsweise fluiddichte Fixierung der HF-Elektrode (8) im Instrumentenkopf (6) mittels zumindest einer Klemmhülse (10), vorzugsweise zweier axial beabstandeter Klemmhülsen erfolgt, die um die HF-Elektrode (8) geführt und stopfenartig in den hülsenförmigen Instrumentenkopf (6) eingesetzt ist/sind.

5. Endoskopisches Chirurgieinstrument nach einem der vorstehenden Ansprüche, **dadurch gekennzeichnet, dass** der hülsenförmige Instrumentenkopf (6) aus einem Keramikmaterial besteht.

6. Endoskopisches Chirurgieinstrument nach einem der vorstehenden Ansprühe, **dadurch gekennzeichnet, dass** die Außen- und Innenhülle (2, 4) aus einem biegeflexiblen Material besteht, das für ein Nachfolgen von Kanalwindungen menschlicher Organe vorzugsweise dem Darmkanal angepasst ist.

7. Endoskopisches Chirurgieinstrument nach einem der vorstehenden Ansprüche, **dadurch gekennzeichnet, dass** der hülsenförmige Instrumentenkopf (6) mit einer Vorschubkraft als Schub- und/Zugkraft über die Innenhülle (4) und/oder die elektrische Zuleitung (16) beaufschlagbar ist, um die HF-Elektrode (8) aus dem distalen Ende der Außenhülle (2) vorzuschieben und/oder in die Außenhülle (2) zurückzuziehen, soweit, dass sie proximal vollständig hinter dem distalen Ende der Außenhülle (2) zu liegen kommt, wobei die Relativbewegung der Außen- und Innenhülle (2, 4) über den Instrumentenkopf (6) gleitgeführt ist.

8. Endoskopisches Chirurgieinstrument nach einem der vorstehenden Ansprüche, **dadurch gekennzeichnet, dass** der hülsenförmige Instrumentenkopf (6) in seinem distalen Endabschnitt außenseitig abgerundet ist.

9. Endoskopisches Chirurgieinstrument nach einem der vorstehenden Ansprüche, **dadurch gekennzeichnet, dass** zumindest die Innenhülle (4) schlauchförmig, vorzugsweise mit einer inneren flexiblen Materialverstärkung ausgebildet ist, um einem Innendruck, verursacht durch das zugeführte Spülmedium, Stand zu halten.

10. Endoskopisches Chirurgieinstrument nach einem der vorstehenden Ansprüche, **dadurch gekennzeichnet, dass** zur Vermeidung von Verkanten des Instrumentenkopfs (6) dieser axial länger ist als der Durchmesser der Außenhülle (2), in welcher er gleitgeführt ist.

## Claims

1. Endoscopic surgical instrument which is adapted to be introduced into a working channel of an endoscope, with
an outside jacket (2), in which an inside jacket (4) is supported relatively displaceable,
a sleeve-shaped instrument head (6) which is formed or fixedly mounted on the distal end section of the inside jacket (4), wherein
the instrument head (6) has a flush channel (12) being open at both axial ends of the instrument head (6),
an RF electrode (8) which is included in an electrically-insulated manner in the instrument head (6), wherein
the electrode (8) has an electrical supply line (16) which is routed within the inside jacket (4), and wherein
the RF electrode (8) together with the instrument head (6) and the electrical supply line (16), is displaceable in an axial direction beyond the distal end of the exterior jacket (2), wherein
the relative position between the RF electrode (8) and an instrument flush medium opening (14) remains constant, wherein the instrument flush-medium outlet opening (14) is directly formed by the at least one flush channel (12) as the most distal one,
**characterized in that**
the RF electrode (8) is immovably fixed in the instrument head (6) in such a way that it basically freely projects in every relative operation position with respect to the outside jacket (2) in the axial direction beyond the distal end of the instrument head (6), and that the instrument head (6) together with the inside jacket (4) in every relative operation position with respect to the outside jacket (2) remains at least partially slidingly guided therein in a fluid-sealed manner, such that the instrument head (6) including the RF electrode (8) and the electrical supply line (16) is displaceable from a position where they are completely enclosed by the outside jacket (2) in an axial direction beyond the distal end of the outside jacket (2) and that the flush channel (12) opens at the proximal front side of the instrument head (6) into the interior compartment of the hose-shaped inside jacket (4), and forms the instrument flush-medium outlet openings (14) at the distal front side of the instrument head (6).

2. Endoscopic surgical instrument according to Claim 1, **characterized in that** the flush channel (12) is subjectable with a flush medium conveyed by the inside jacket (4).

3. Endoscopic surgical instrument according to Claim 1 or 2, **characterized in that** the sleeve-shaped instrument head (6) has a distal axial section (6a) with large outer diameter, which is located directly at the outside jacket (2) in a sliding manner and has a proximal axial section (6 b) with small outside diameter as a fluid-sealing insertion or screw-on base socket for the inside jacket (4).

4. Endoscopic surgical instrument according to one of the above Claims, **characterized in that** the preferably fluid-sealed fixing of the RF electrode (8) within the instrument head (6) is implemented by means of at least one clamp bushing (10), preferably two axially-offset clamp bushings, that is/are located around the RF electrode (8) and is/are fitted plug-like in the sleeve-shaped instrument head (6).

5. Endoscopic surgical instrument according to one of the above Claims, **characterized in that** the sleeve-shaped instrument head (6) consists of a ceramic material.

6. Endoscopic surgical instrument according to one of the above Claims, **characterized in that** the outside and inside jackets (2, 4) consist of a bend-flexible material which is adapted to follow channel turns in human organs, preferably the intestine.

7. Endoscopic surgical instrument according to one of the above Claims, **characterized in that** the sleeve-shaped instrument head (6) is subjected or subjectable to an advancing force as a push- and pull-out force via the inside jacket (4) and/or the electrical supply line (16), in order to advance the RF electrode (8) from the distal end of the outside jacket (2) and/or to withdraw it into the outside jacket (2), to such an extent that it comes to rest completely proximal behind the distal end of the outside sleeve (2), wherein the relative movement of the outside and inside sleeves (2, 4) is slidingly guided by the instrument head (6).

8. Endoscopic surgical instrument according to one of the above Claims, **characterized in that** the sleeve-shaped instrument head (6) is rounded off in its distal end section on its outside.

9. Endoscopic surgical instrument according to one of the above Claims, **characterized in that** at least the inside jacket (4) is formed hose-shaped, preferably with an inner flexible material reinforcement, in order to resist an internal pressure caused by the supplied flush medium.

10. Endoscopic surgical instrument according to one of the above Claims, **characterized in that**, to avoid canting of the instrument head (6), this is axially longer than the diameter of the outside jacket (2) in which it is slid.

## Revendications

1. Instrument chirurgical endoscopique, qui est adapté pour être introduit dans un canal de travail d'un endoscope, comprenant :
une enveloppe extérieure (2), dans laquelle une enveloppe intérieure (4) est montée de manière à pouvoir être coulissée de manière relative,
une tête d'instrument (6) présentant une forme de douille, qui est réalisée ou est montée de manière solidaire au niveau de la section d'extrémité distale de l'enveloppe intérieure (4), dans lequel
la tête d'instrument (6) présente un canal de rinçage (12), qui s'ouvre au niveau de deux extrémités axiales de la tête d'instrument (6),
une électrode HF (8), qui est logée de façon électriquement isolée dans la tête d'instrument (6), dans lequel
l'électrode (8) présente une ligne d'alimentation (16) électrique, qui est placée en longueur à l'intérieur de l'enveloppe intérieure (4) et dans lequel
l'électrode HF (8) peut être déplacée par coulissement dans la direction axiale au-delà de l'extrémité distale de l'enveloppe intérieure (2) conjointement avec la tête d'instrument (6) et avec la ligne d'alimentation (16) électrique, dans lequel
la position relative entre l'électrode HF (8) et une dernière ouverture de sortie de milieu de rinçage d'instrument (14) distale, formée directement par l'au moins un canal de rinçage (12), reste constante,
**caractérisé en ce**
**que** l'électrode HF (8) est fixée de manière immobile dans la tête d'instrument (6) de telle manière qu'elle dépasse librement en principe dans la direction axiale de l'extrémité distale de la tête d'instrument (6) dans chaque position de fonctionnement relative par rapport à l'enveloppe extérieure (2), et en ce que la tête d'instrument (6) demeure, conjointement avec l'enveloppe intérieure (4), dans chaque position de fonctionnement relative par rapport à l'enveloppe extérieure (2), guidée par glissement de manière étanche aux fluides au moins en partie dans cette dernière de telle manière que la tête d'instrument (6), y compris l'électrode HF (8) et la ligne d'alimentation (16) électrique, peut être déplacée par coulissement dans la direction axiale hors d'une position entourée en totalité par l'enveloppe extérieure (2) au-delà de l'extrémité distale de l'enveloppe extérieure (2), et en ce
**que** le canal de rinçage (12) s'ouvre au niveau du côté frontal proximal de la tête d'instrument (6) dans l'espace intérieur de l'enveloppe intérieure (4) de forme tubulaire et forme, au niveau du côté frontal distal de la tête d'instrument (6), l'ouverture de sortie de milieu de rinçage (14) de l'instrument.

2. Instrument chirurgical endoscopique selon la revendication 1, **caractérisé en ce**
**que** le canal de rinçage (12) peut être soumis à l'action d'un milieu de rinçage transporté à travers l'enveloppe intérieure (4).

3. Instrument chirurgical endoscopique selon la revendication 1 ou 2, **caractérisé en ce que** la tête d'instrument (6) présentant une forme de douille a une section axiale (6a) distale présentant un diamètre extérieur de grande taille, laquelle est en appui par glissement directement au niveau de l'enveloppe extérieure (2) et a une section axiale (6b) proximale présentant un diamètre extérieur de petite taille en tant que socle d'emboîtement ou d'enfilage par vissage assurant l'étanchéité aux fluides pour l'enveloppe intérieure (4).

4. Instrument chirurgical endoscopique selon l'une quelconque des revendications précédentes, **caractérisé en ce que** la fixation de préférence étanche aux fluides de l'électrode HF (8) dans la tête d'instrument (6) est effectuée au moyen au moins d'une douille de serrage (10), de préférence au moyen de deux douilles de serrage espacées axialement, laquelle/lesquelles est/sont guidée/guidées autour de l'électrode HF (8) et est/sont insérée/insérées à la manière d'un bouchon dans la tête d'instrument (6) présentant une forme de douille.

5. Instrument chirurgical endoscopique selon l'une quelconque des revendications précédentes, **caractérisé en ce que** la tête d'instrument (6) présentant une forme de douille est constituée d'un matériau en céramique.

6. Instrument chirurgical endoscopique selon l'une quelconque des revendications précédentes, **caractérisé en ce que** l'enveloppe extérieure et l'enveloppe intérieure (2, 4) sont constituées d'un matériau flexible, qui est adapté pour épouser des convolutions de canal d'organes humains, de préférence pour épouser le canal intestinal.

7. Instrument chirurgical endoscopique selon l'une quelconque des revendications précédentes, **caractérisé en ce que** la tête d'instrument (6) présentant une forme de douille peut être soumise à l'action d'une force d'avancée faisant office de force de poussée et/ou de traction par l'intermédiaire de l'enveloppe intérieure (4) et/ou de la ligne d'alimentation (16) électrique afin d'avancer l'électrode HF (8) hors de l'extrémité distale de l'enveloppe extérieure (2) et/ou de la ramener dans l'enveloppe extérieure (2) jusqu'à ce qu'elle parvienne en appui de manière proximale totalement derrière l'extrémité distale de l'enveloppe extérieure (2), dans lequel le déplacement relatif de l'enveloppe extérieure et intérieure (2, 4) est guidé par glissement par l'intermédiaire de la tête d'instrument (6).

8. Instrument chirurgical endoscopique selon l'une quelconque des revendications précédentes, **caractérisé en ce que** la tête d'instrument (6) présentant une forme de douille est arrondie côté extérieur dans sa section d'extrémité distale.

9. Instrument chirurgical endoscopique selon l'une quelconque des revendications précédentes, **caractérisé en ce qu'**au moins l'enveloppe intérieure (4) est réalisée de manière à présenter une forme tubulaire, de préférence avec un renforcement de matériau flexible intérieur afin de faire face à une pression intérieure provoquée par le milieu de rinçage amené.

10. Instrument chirurgical endoscopique selon l'une quelconque des revendications précédentes, **caractérisé en ce que** la longueur axiale de la tête d'instrument (6) est plus grande que le diamètre de l'enveloppe extérieure (2), dans laquelle elle est guidée par glissement, afin d'éviter le gauchissement de ladite tête d'instrument.
